(19) Europäisches Patentamt
European Patent Office
Office européen des brevets

(11) Publication number: **0 061 312**
**B1**

(12) **EUROPEAN PATENT SPECIFICATION**

(45) Date of publication of patent specification: **18.06.86**

(51) Int. Cl.$^4$: **A 61 L 17/00,** A 61 F 2/00, C 08 J 7/12

(21) Application number: **82301408.9**

(22) Date of filing: **18.03.82**

(54) **Alkyl-substituted polymers having enhanced albumin affinity.**

(30) Priority: **19.03.81 US 245259**

(43) Date of publication of application:
**29.09.82 Bulletin 82/39**

(45) Publication of the grant of the patent:
**18.06.86 Bulletin 86/25**

(84) Designated Contracting States:
**BE DE FR GB NL SE**

(58) References cited:
**GB-A-1 136 669**
**US-A-3 051 588**
**US-A-3 755 218**
**US-A-3 839 743**
**US-A-4 046 725**

(73) Proprietor: **BOARD OF REGENTS
THE UNIVERSITY OF TEXAS SYSTEM
201 West 7th Street
Austin Texas 78701 (US)**

(72) Inventor: **Munro, Mark S.
Route 1
Anna Texas 75003 (US)**
Inventor: **Quattrone, Alfred J.
2338 Stromcroft Court
Westlake California 91361 (US)**
Inventor: **Ellsworth, Steven R.
2537 Rampart
Dallas Texas 75235 (US)**
Inventor: **Eberhart, Robert C.
10519 Royal Springs
Dallas Texas 75235 (US)**

(74) Representative: **Brown, John David et al
FORRESTER & BOEHMERT
Widenmayerstrasse 4/I
D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 061 312 B1**

**Description**

The present invention relates generally to methods of preparing materials for biomedical application and more specifically to methods of preparing biomaterials having an enhanced affinity toward albumin.

In the application, implementation and implantation of biomaterials in bodily tissues, the problem of biocompatibility and biofunctionality of these materials has been the subject of extensive investigation. In particular those biomaterials which are intended to contact body tissues over a long period of time present various problems associated with physiological and chemical stability and compatability with respect to various contacted tissues.

Both bulk and surface properties determine the functional biocompatibility of the material. Mechanical strength, elasticity, flexibility, creep and fatigue resistance, chemical inertness, impermeability to water vapor, resistance to acid attack, etc. are desired bulk properties of many biomaterials which should be maintained in vivo. The surfaces of exogenous materials in contact with bodily tissues should desirably exhibit resistance to red and white thrombus formation (e.g. blood coagulation, platelet adhesion and aggregation) immunological attack, cell adhesion, panus formation, etc. Thrombogenesis, embolization, pannus formation, etc. resulting from blood and other tissue interacting with the surface may compromise the intended use of the biomaterial in certain medical devices, and quite possibly result in device failure.

Application of most non-physiologic biomaterials and protheses to tissue contact initiates a series of physiologic events on the surface of such biomaterials. In particular, a biomaterial such as a synthetic polymer in contact with blood rapidly forms an adsorbed protein layer. Within seconds after application, the biomaterial interface is coated with a thin proteinaceous film, rich in fibrinogen, fibronectin and gamma globulin. As blood circulates, further protein components contribute to the thickness of the film. Conformational alterations and complexing of proteins occur, activating defense mechanisms, e.g. coagulation, platelet adhesion and aggregation, white cell adhesion, etc.

A number of approaches to provide tissue compatibility and specifically blood thromboresistance have been proposed and many promising materials have been developed. However none of the biomaterials developed heretofore have been totally successful and most have provided a poor compromise between device function and long term compatibility.

One such approach, has been to modify the surface of existing biomaterials in an effort to prevent endogenous protein adhesion and accumulation. Surface modification techniques which have been evaluated for biocompatibility and functionality include low polarity surfaces, negatively charged surfaces, and surfaces coated with biological material, e.g. enzymes, endothelial cells and proteins. One example of this type of approach is the provision of fluoroalkyl side chained organic polymeric material simultaneously having low surface tension and negative zeta potential (see US—A—3 839 743).

The low polarity surfaces such as silicone polymers, and hydrogels, were developed in the view that low surface free energy, specifically low interfacial energy would limit the driving potential for adhesion of proteins and cellular material. Although the silicone biomaterials are substantially chemically inert and improve blood compatibility, platelet aggregation and cell accumulation eventually result with blood contact, especially at low blood flow rates.

Another approach to enhance thromboresistance was to provide materials having negatively charged surfaces. Electrets, hydrogels and negatively charged biological molecules such as heparin, exhibit this property and appear to have improved, but not provided complete thromboresistance. Hydrogels, water saturated polymeric gels exhibiting a net negative surface charge, offer high biological compatibility but by their very nature of high water content lack structural strength and durability.

The biological coated polymers are of considerable interest due to their variability and complexity. Proteinaceous material such as heparin, albumin, and streptokinase have all been covalently bound to polymeric surfaces to enhance thromboresistance. Albumin is of particular interest for a surface coating because of its apparent passivating activity.

Heretofore, albumin has been physically adsorbed, and electrostatically and covalently bound to polymer surfaces. While temporary and partial protection against thrombogenesis is obtained by these methods, the albumin coating is eventually denatured or lost. The loss of albumin functionality when passively adsorbed may be traced to competitive reactions with other proteins having higher affinities for the polymer surface, ablation of the adsorbed albumin, or conformational changes, and fragmentation. Furthermore covalently bound albumin is subject to internal masking by the polymer tertiary structure caused by long term reconstitution of the polymer surface. As the polymer undergoes tertiary reorganization new, unfilled binding sites are presented to which thromogenic proteins may gain a "foothold".

It is therefore highly desirable, and a primary object of this invention to provide polymeric surfaces which are biocompatible and are functional over a long period of time. It is further an object of this invention to provide consistent thromoresistance, resistance to cell adhesion in general and resistance to immunological attack over a range of blood flow rates including stasis, pH, electrolyte conditions, and hematologic makeups such as anemia, polycythemia, and thrombocytemia.

According to the first aspect of the present invention, there is provided a biocompatible, nonthrombogenic polymeric material, characterized in that it comprises a polymeric substrate having

covalently attached thereon aliphatic extensions having from 14 to 30 carbon atoms, the extensions providing selective, reversible affinity-binding sites for albumin.

According to the second aspect of the present invention there is provided a device adapted for use in contact with blood components containing albumin, characterized in that the device has disposed on the surfaces thereof which contact blood components a polymeric material in accordance with the first aspect.

According to the third aspect of the present invention there is provided a vacular graft comprising a tubular conduit, characterized in that the tubular conduit is constructed of a polymeric material in accordance with the first aspect.

According to the fourth aspect of the present invention there is provided a method of minimizing thrombus formation on the surfaces of a device adapted for use in the presence of blood components containing albumin, characterized in that the method comprises the step of providing a layer of polymeric material in accordance with the first aspect on the surface of the device at the area of contact between the blood components and the device.

The aliphatic extensions, preferably have from 14 to 18 carbon atoms and are also preferably straight-chained alkyl groups, especially n-octadecyl groups.

Thus, the invention relates to polymeric biomaterials having covalently bound onto the polymer surface extended aliphatic chains. In physiologic application, these extended aliphatic chains provide high affinity, reversible hydrophobic binding sites for albumin. Materials comprising the polymers of the present invention offer substantially improved surface binding of albumin, the major serum protein, relative to the other serum proteins. Selective adsorption of albumin to the exclusion of thrombogenic proteins is accomplished by exploiting the strong affinity albumin has for $C_{14}$ to $C_{30}$, preferably $C_{14}$ to $C_{18}$, aliphatic moieties.

By establishing an albumin coating on biomaterials through selectively increasing the affinity of the surface for albumin, the problems of the prior art, e.g. passively absorbed endogenous albumin and covalently bound exogenous albumin are eliminated. The products of this invention provide a binding site with enhanced affinity for endogenous albumin. As the native albumin denatures or desorbs at the aliphatic binding site, new albumin molecules favorably compete for replacement to the exclusion of other proteins. A dynamic, selective process is set up whereby albumin preferentially occupies the exposed binding sites by virtue of albumin's high concentration in the blood and the enhanced affinity provided by the aliphatic chains. More significantly, since many binding sites have been occupied and/or masked by the albumin-aliphatic chain complex, those proteins implicated in thrombus formation are inhibited from binding to the polymer surface. Thus, the high albumin affinity polymeric biomaterial, with its biologically functional, renewable albumin coat can maintain thromboresistance indefinitely.

In addition to the composition embodiments, this invention contemplates five methods of covalently binding aliphatic chains to polymer surfaces.

The first method employs a two-step substitution reaction whereby polymers with active hydrogens such as amides or alcohols are deprotonated by an aprotonous base thereby forming the corresponding amide ion or oxide ion intermediate. Subsequent treatment with an aliphatic halide yields a substituted amide or ether.

The second method of polymer surface substitution is the direct reaction between an aliphatic isocyanate and active sites on the polymer surfaces. The electron rich isocyanate moiety functions both as the proton abstracting base and the substituting nucleophilic reagent.

A third method of preparation contemplates a substitution reaction between a primary amine and a carbonyl function of a polymer. A proton abstracting base may also be employed to facilitate the reaction. This reaction results in the conversion of the carbonyl group into an alkyl-substituted amide group.

A fourth method contemplates polymer reaction with a diisocyanate to yield a highly reactive monoisocyanate intermediate bound to the polymer surface. This monoisocyanate then undergoes subsequent reactions with alkyl alcohols, amines or acids, yielding an alkyl substitution onto the polymer.

A fifth method of preparation contemplates derivitization based on a polyester monomer, 2-nitro-1,4, dimethylbenzene replacing p-xylene. This material is oxidized, to form 2-nitro-terephthalic acid. The nitro group is reduced to an amino group and protected. This material is then polymerized. After synthesis the amino group is reacted with an alkyl compound to give the substituted polymer.

The production of polymers having aliphatic chains covalently bound to the polymer surface is of considerable importance to the biomedical field in the application of biomaterials. The surfaces of biomaterials comprised of aliphatic substituted polymers exhibit improved thromboresistance and tissue compatibility without loss of functionality and stability. Further these products present minimal derivitization and disruption of polymer composition thereby avoiding the enhanced immunogenicity associated with biomaterials having foreign protein material directly attached to the polymer surface.

Moreover, the importance of this invention lies in the potential longevity of this blood compatible, modified polymer surface in long term implant applications. Further, the products of this invention overcome the problems associated with static coating of protein layers covalently linked to polymer surfaces. The earlier, covalently coated polymers have yielded their thromboresistance potential to such problems as denaturation during fixation to the polymer surface, loss of the protein coat by viscous drag in a flow field, serum protease degradation, and teriary redistribution. The aliphatic side chain substituted

polymers described herein, however, are not susceptible to these problems, but rather provide for dynamic replacement of albumin from blood or serum itself.

Embodiments are discussed wherein various types of polymers, presently implemented as biomaterials for implants and tissue contact, are employed as substrates for the attachment of aliphatic chain groups. The aliphatic chain groups are preferably selected from various alkyl compounds having 14 to 30 carbon atoms, and most preferably having 14 to 18 carbon atoms.

This invention relates to polymeric materials having selective enhancement toward albumin affinity. These materials are effective, in selectively binding albumin to the exposed material surface, thereby preventing subsequent thrombus formation and cell adhesion. Since the albumin binding is a dynamic process, the nonthrombogenic and surface passivating features of the materials are of indefinite long term duration.

The following discussion is in terms of the preferred embodiments of this invention, which represent the best mode known to the inventors at the time of this application.

In accordance with the preferred embodiments of this invention, a suitable polymer is provided in its solid or semi-solid state. Suitable polymers for use in this reaction are those polymers possessing active surface hydrogens. An active surface hydrogen is a hydrogen that may be removed by a proton-abstracting base without substantial disruption of the polymer backbone. Examples of polymers exhibiting active surface hydrogens include but are not limited to polyurethanes, polyesters, polyamides, polyamines, polyimines, polyacrylamides, polystyrenes and celluloses.

Selection of the polymeric substrate will vary according to the functional needs of the biomaterial device. For example, to prevent thrombus formation on dialysis tubing or membranes, the processes of this invention are applied to cellulose membranes without substantially altering the membranes' original dialysis specifications. Indeed most biomaterials in wide application today are amenable to the processes of this invention.

Further, these polymers have no limitations as to size, shape, or form in which they may be utilized. The polymers may be subject to the substitution reactions of this invention before or after they are molded or extruded into their final form. In this regard, thin films, sheets, membranes, tubes, hollow fibers, and particulate matter are a few forms which are suitable for use in the present invention.

Further in accordance with such embodiments of the present invention, the polymer is exposed to a proton abstracting base to yield an activated polymeric surface. Suitable proton abstracting bases include but are not limited to sodium ethoxide, sodium butylate, potassium or sodium hydride, methyl magnesium bromide, and various isocyanates and diisocyanates.

Further in accordance with the preferred embodiments of this invention, chemical attachment of $C_{14}$ to $C_{30}$ aliphatic chains to the polymer backbone is accomplished by exposing the activated polymer surface to a solution of the desired alkyl reactants comprising functional group monomers such as alkyl halids, alkyl tosylate, alkyl methylsulfonate, alkyl isocyanate, or alkyl monoamine. The reaction is desirably accomplished in a non-proton donating solvent such as toluene, hexane, dioxane, diethyl ether or trimethylpentane.

The exposure times required to effect the substitution reaction will depend on the polymer substrate, the intermediate bifunctional molecule and/or the alkyl monomer selected, and the desired extent of substitution. Generally, narrow ranges of the concentrations of reactants and exposure times are required to produce the greatest degree of polymer substitution. Further the concentration of reactants and the extent of exposure time will affect penetration of the polymer substrate.

Alternatively, polymers substantially without available surface active hydrogens, such as polyesters, are also amenable to the substitution reactions of this invention. Instead of abstracting a hydrogen from the polymer surface, the proton abstraction process abstracts the active hydrogen from the alkyl monomer. For example, substitution reactions employing alkyl monoamines and polyester substrates involve proton abstraction from the primary amine function followed by lysis of a polyester function resulting in an alkyl-substituted amide group along the polyester chain.

Alternatively, polymers containing reactive surface sites may also be substituted via reaction with alkyl isocyanates. In this reaction scheme, the alkyl monomer may add directly into the polymer chain without formation of a reactive intermediate.

Moreover, a bifunctional isocyanate group may initially be substituted into the polymer chain with subsequent binding of a suitable alkyl group. For example, substitution reactions with diisocyanates followed by treatment with alkyl alcohols or acids yields alkyl substituted polymeric chains.

Even further, monomeric polymer precursors may be synthesized such that they possess alkyl side chains which may function as high affinity binding sites for albumin. Following polymerization these groups will be available, without post-polymerization modification, for albumin binding.

According to the processes of this invention, reaction products are formed comprising a polymeric substrate having covalently bound thereon aliphatic chain functional groups having 14 to 30, preferably 14 to 18 carbon residues. In physiologic application, the aliphatic functional groups serve to selectively and reversibly bind albumin. The albumin bound to the polymer surface forms an effective, dynamic nonthrombogenic coating on the biomaterial.

The reaction schemes between the various representative polymers widely in use for biomedical application and representative aliphatic functional group monomers are as follows:

Reaction Scheme I

A reaction scheme is presented below between polyurethanes and RY wherein RY represents an alkyl halide or alkyl sulfonate having 14 to 30 carbon residues. Examples of the leaving group Y are bromide, fluoride, chloride, iodide, tosylate, methylsulfonate and the like.

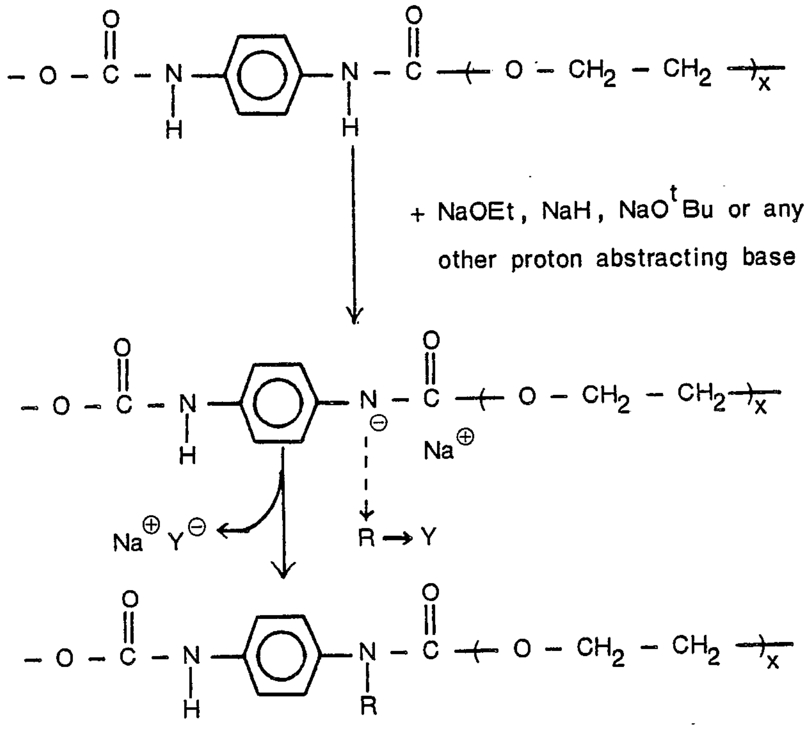


Reaction Scheme II

A reaction scheme is depicted below illustrating nucleophilic substitution between polyamides and RY, wherein RY represents an alkyl halide or alkyl sulfonate.

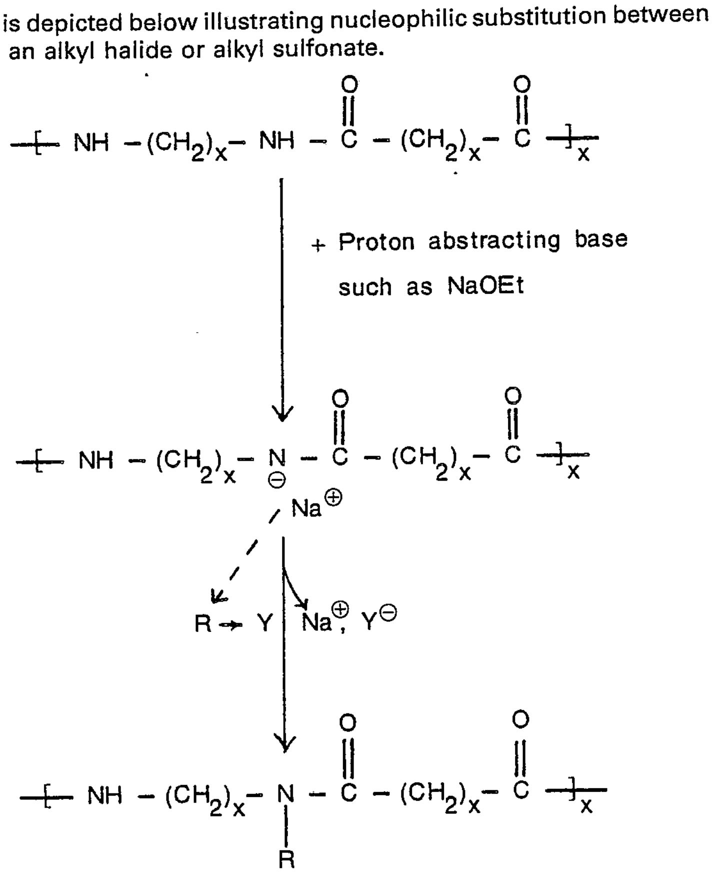


Alkyl-Substituted Polyamide at an Amide Group

Reaction Scheme III

A reaction scheme between polyesters and RY, wherein RY represents an alkyl halide or alkyl sulfonate, is represented as follows:

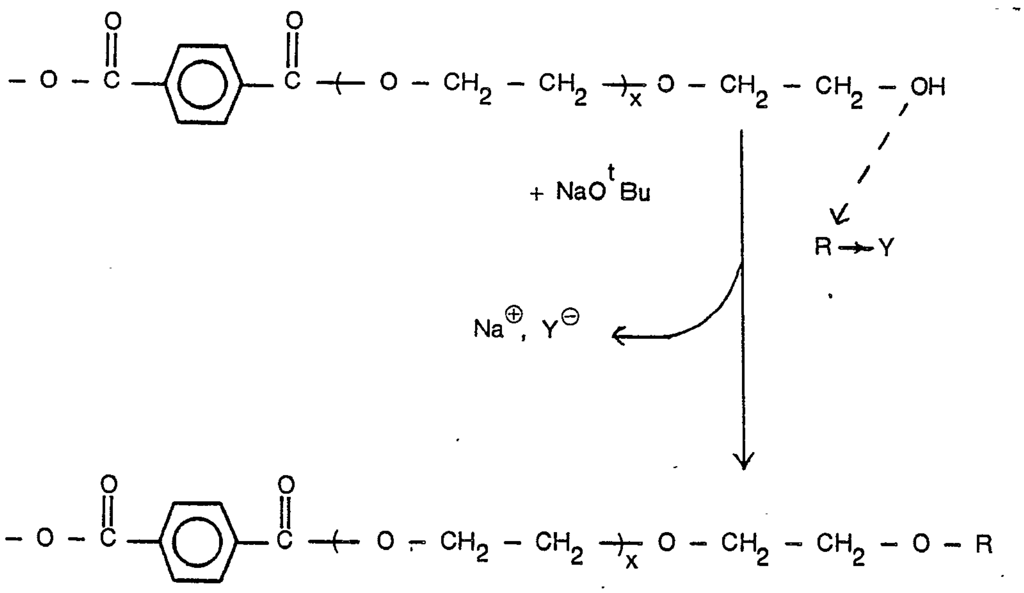


Alkyl-Substituted Polyester at a Primary Alcohol Group forming an Alkyl Ether

Reaction Scheme IV

Reaction scheme between polyacrylamides and RY is represented as follows:

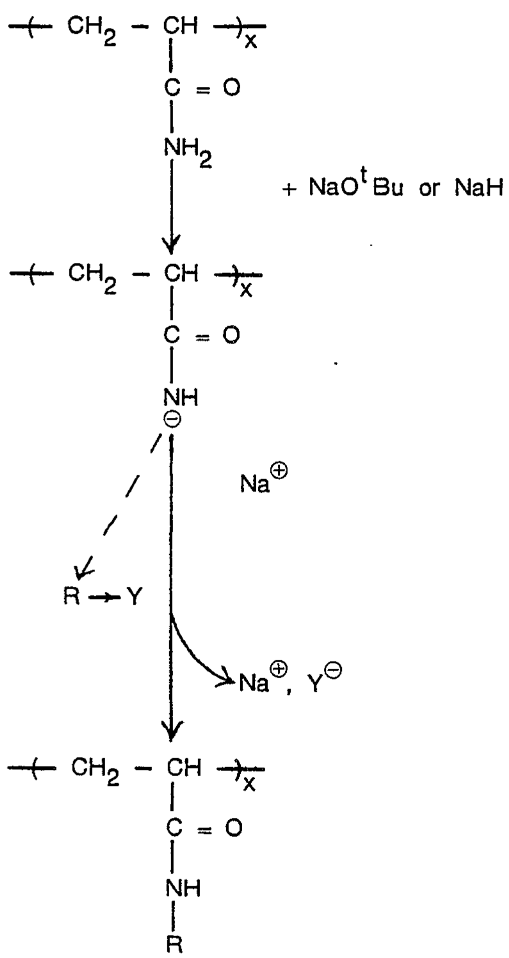


Alkyl-Substituted Polyacrylamide with Formation of Alkyl-Secondary Amide Groups

Reaction Scheme V

A reaction scheme is depicted below between polyurethanes and alkyl isocyanates, R—N=C=O, wherein R represents an alkyl having 14 to 30 carbon residues. The reaction may proceed at a urethane secondary amide group or at an exposed primary alcohol residue.

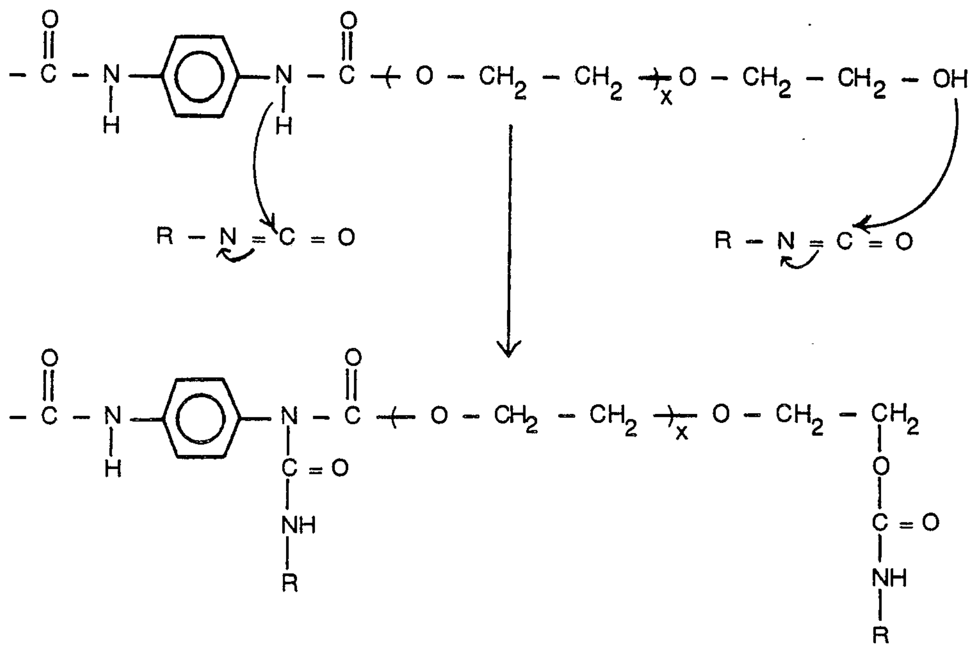

Reaction Scheme VI

Reaction schemes between polyamides and alkyl.isocyanates are depicted below involving both the amide and terminal amine groups of the polyamides.

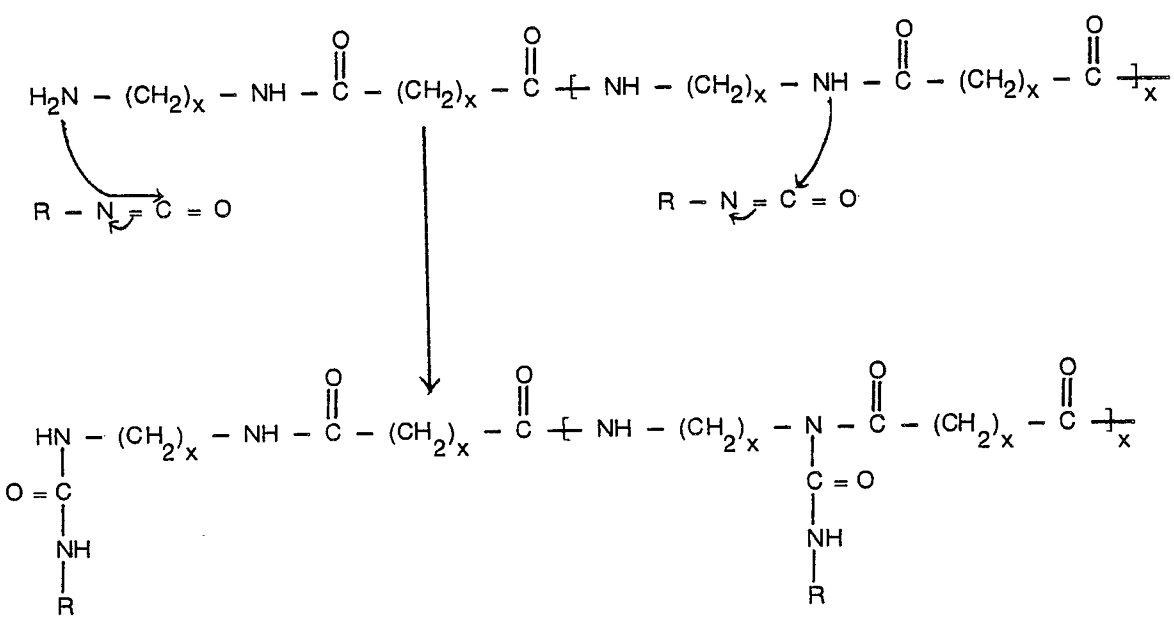

Alkyl-Substituted Urea Group

Reaction Scheme VII

A reaction scheme is shown below between polyesters and alkyl isocyanates, R—N=C=O, where R is an alkyl having 14 to 30 carbon residues.

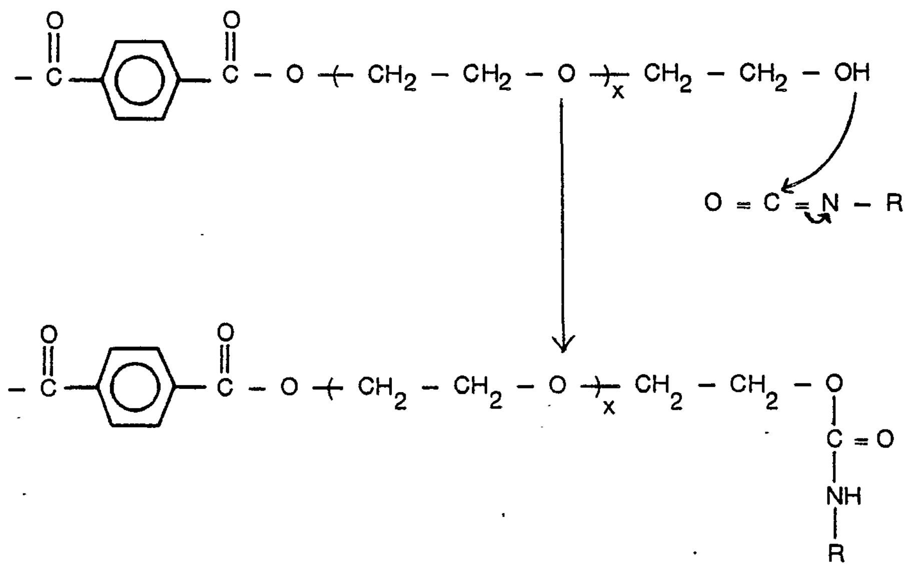

A Terminal Alkyl-Substituted Urethane Group

Reaction Scheme VIII

A reaction scheme is presented below between polyacrylamides and alkyl isocyanates, R—N=C=O, wherein R is an alkyl having 14 to 30 carbon residues.

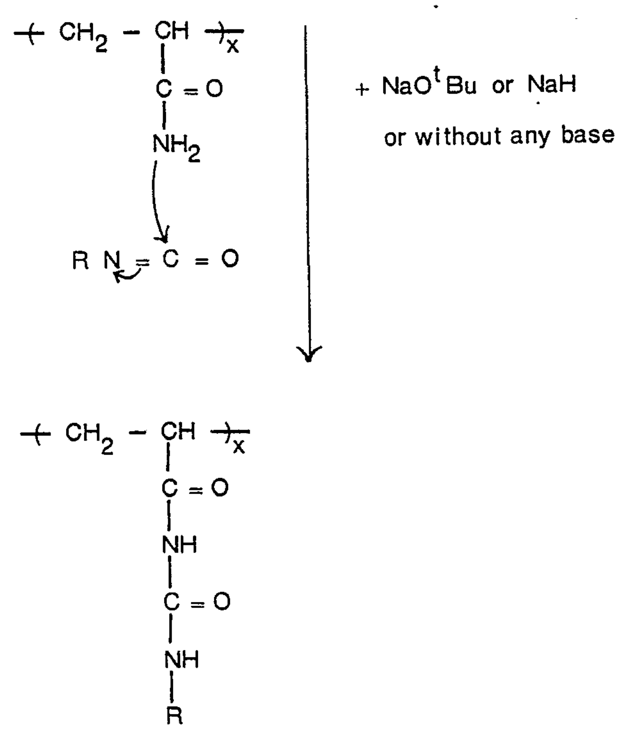

Reaction Scheme IX

A reaction scheme is shown below between a carbonyl function of polyester and alkyl amines R N $H_2$ wherein R represents an alkyl having 14 to 30 carbon residues. The reaction is enhanced in the presence of a proton abstracting base.

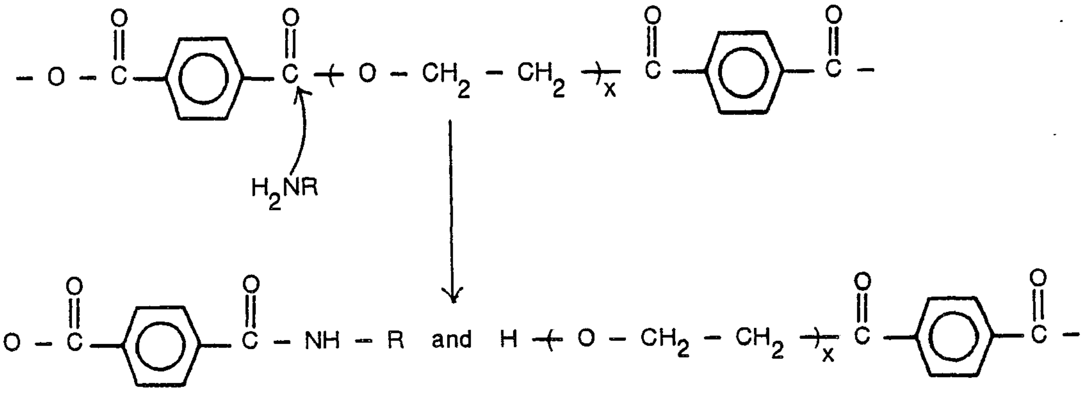

Conversion of an Ester Group to an Alkyl-Substituted Amide Group

Reaction Scheme X

Surface groups such as amines and alcohols on polyurethanes, polyamides and polyacrylamides, and other similar polymers, can be reacted with a bifunctional reactant such as diisocyanate to yield a highly reactive monoisocyanate intermediate bound to the surface. This monoisocyanate may then undergo subsequent reaction with alkyl alcohols, amines or acids, with the net effect of giving an alkyl substitution onto the polymer.

A typical reaction series is shown below:

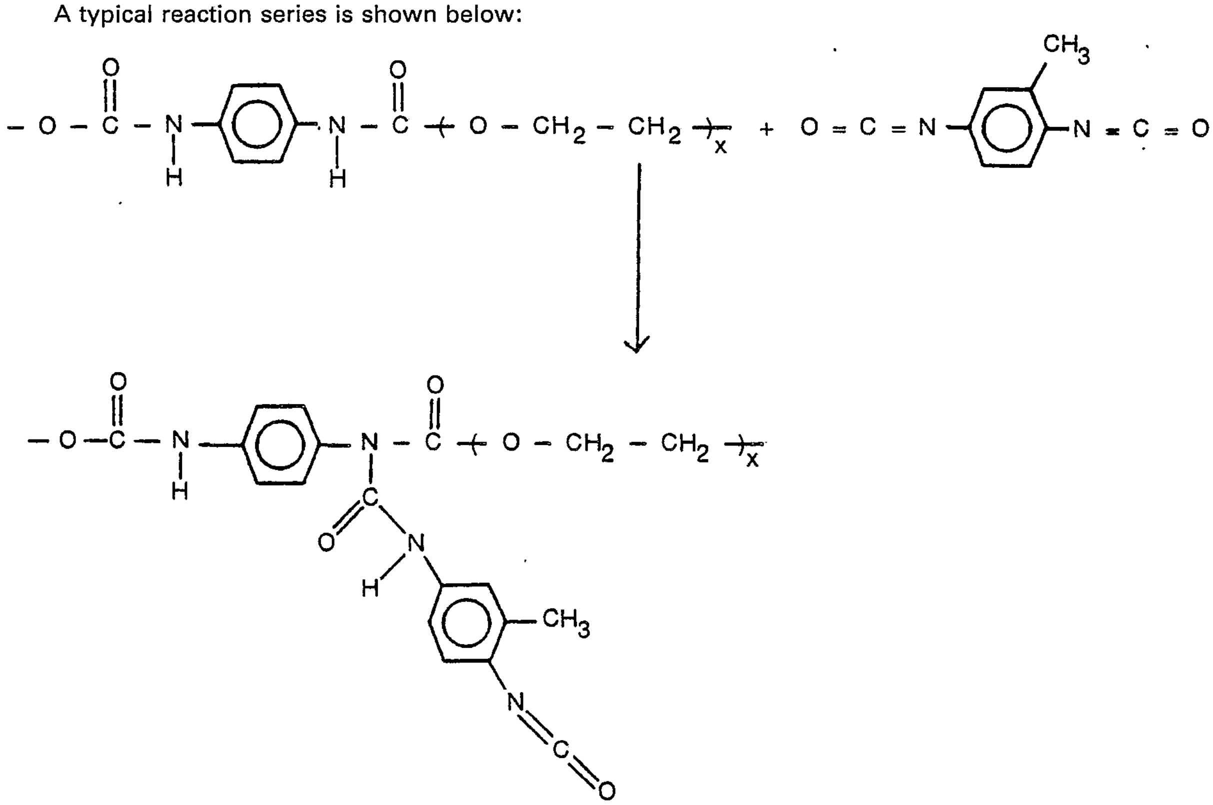

Reactive Monoisocyanate Intermediate

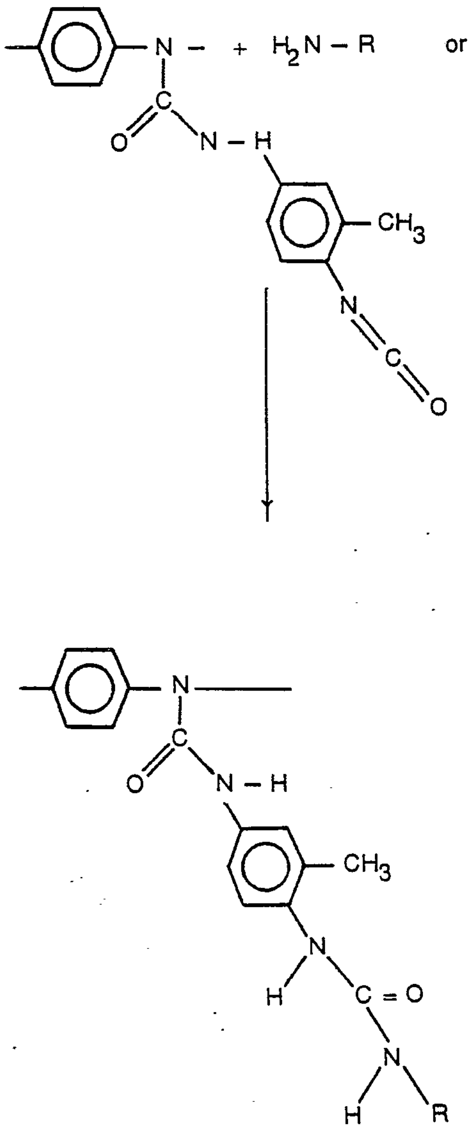

or

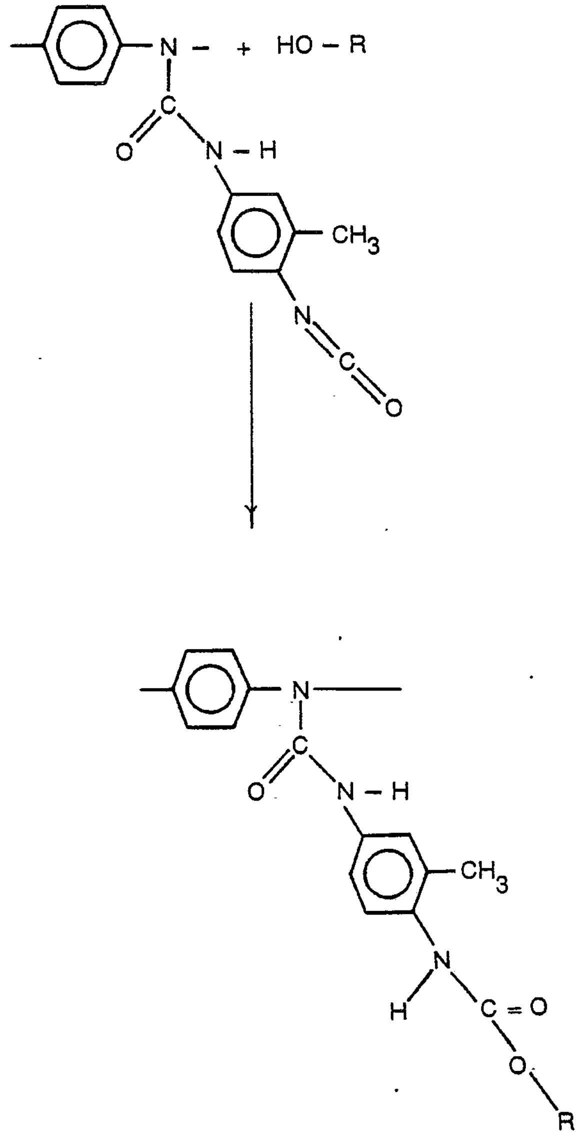

Wherein R is an alkyl chain having 14—30 carbon residues, and preferably 14—18 carbon residues

Diisocyanate substituted residues may occur at any exposed primary or secondary terminating amines or alcohols

A suitable diisocyanate is 2,4 toluene diisocyanate which can be formed from 2,4 diaminotoluene (Aldrich) and excess phosgene with subsequent heat treatment at 115°C.

Reaction Scheme XI

As a starting material for polyester synthesis, 2-nitro-1,4 dimethylbenzene is used in place of p-xylene. This material is oxidized to form 2-nitro-terephthalic acid. The nitro group is then reduced to an amino group and protected, as with trifluoroacetic anhydride. This material is then polymerized, as in typical polyester synthesis. After synthesis the amino group is then reacted with a fatty acid under standard conditions to give the substituted polymer.

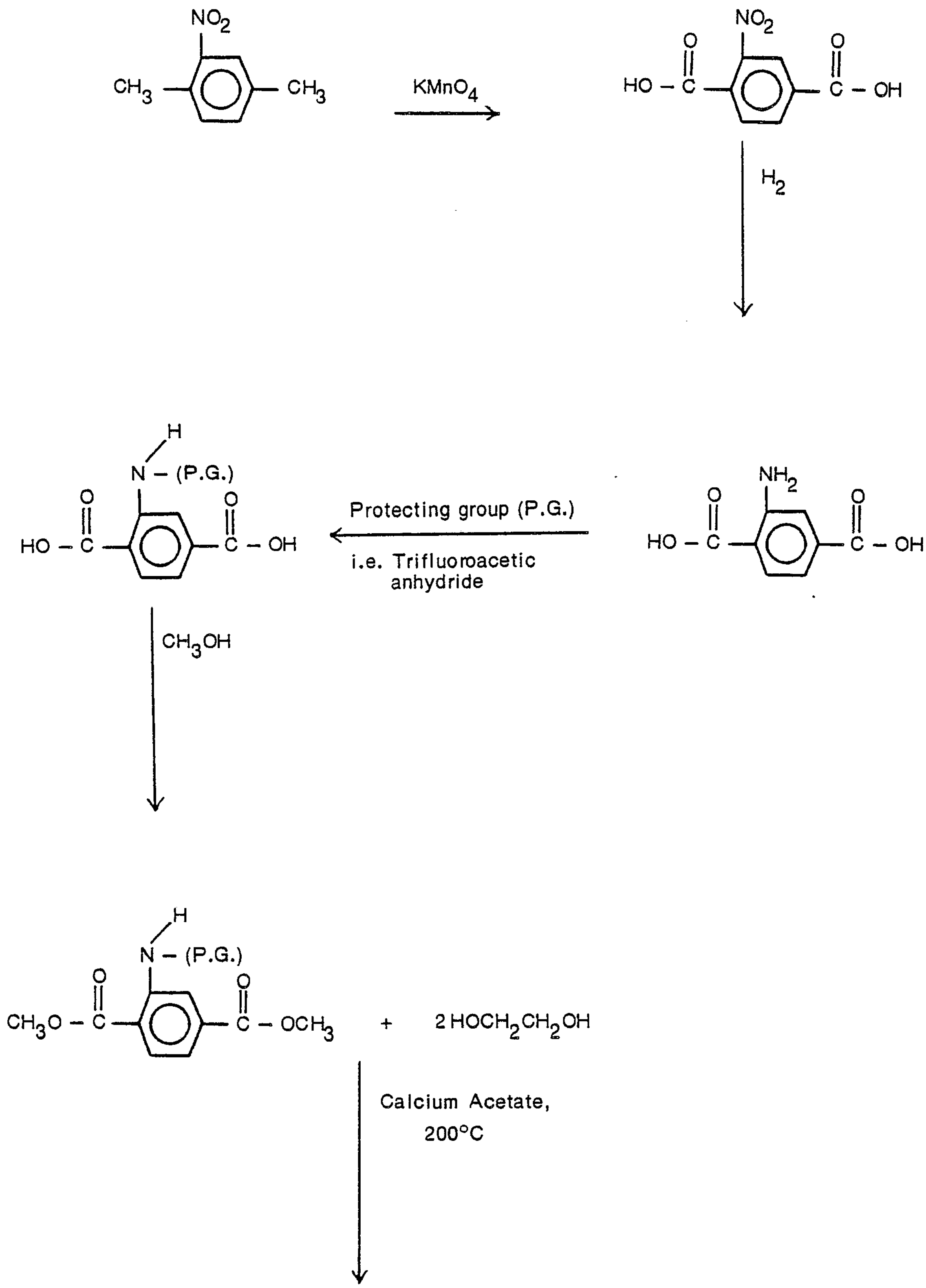

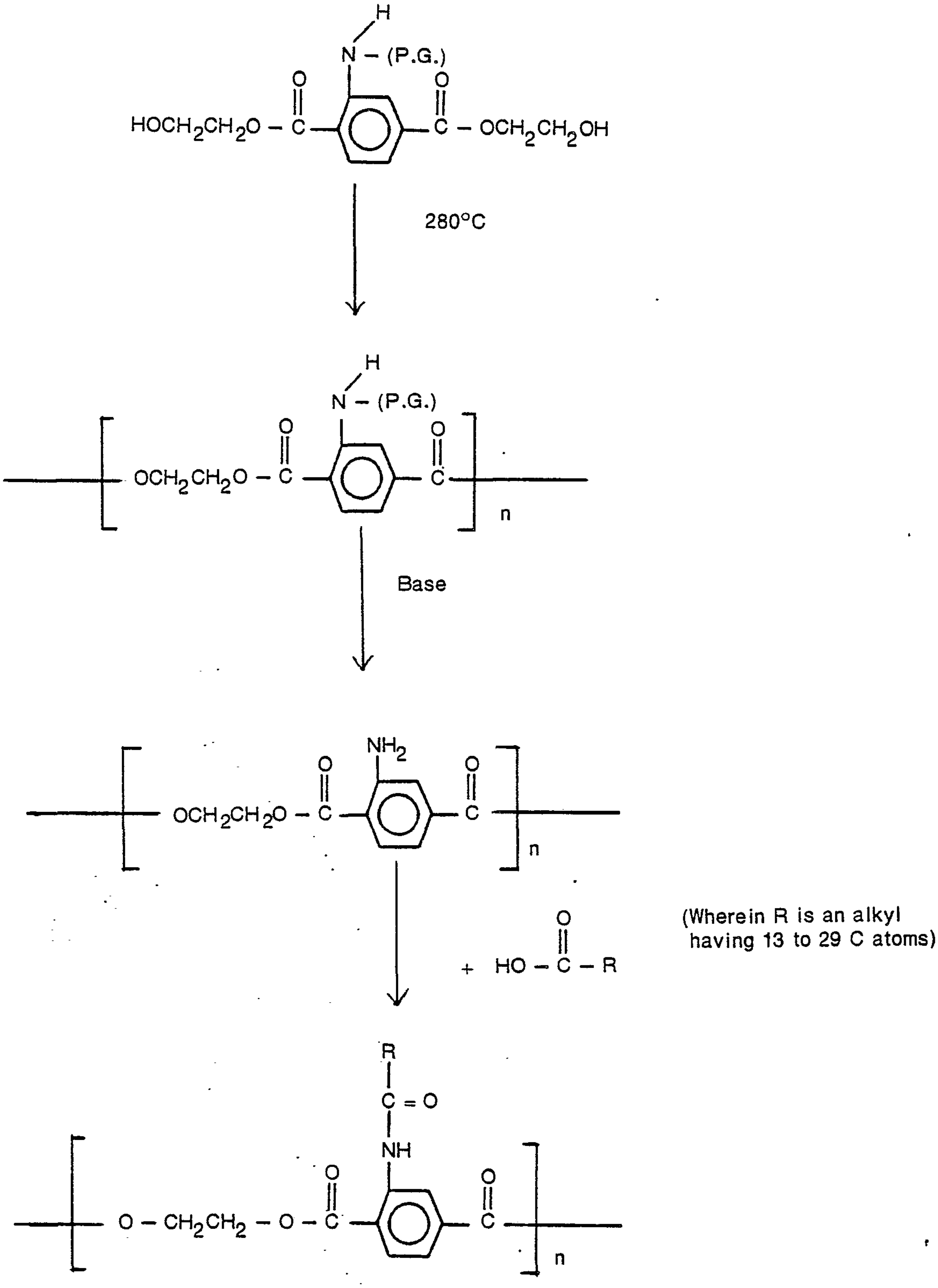


Evaluation and measurement of enhanced albumin affinity to the various alkyl-substituted polymers as compared to non-substituted polymers were carried out as follows. Polymer samples were first immersed in degassed phosphate buffered saline solution, pH 7.4, for more than 15 minutes to suitably hydrate the samples. A selected volume of albumin solution spiked with radiolabelled $^{125}$I-albumin as a tracer was mixed in the sample solution. The resulting albumin solution comprised a concentration of 12—50 mg albumin/dl in phosphate buffered saline. Crystalline human albumin, 99% pure, is available from Miles Laboratories and U.S. Biochemical Corporation. Albumin was radiolabelled according to a modified general technique of a globulin labelling procedure as described by Fraker and Speck, 80 *Biochem. Biophys. Res. Comm.* 849 (1978).

A twofold wash with phosphate buffered saline was conducted at the end of a 30 second $^{125}$I-albumin exposure period. Care was taken to ensure the sample remained well below the air-solution interface. Following the wash step, samples were transferred to counting vials and were counted for 5 minutes in a well-type scintillation counter (Tracor Analytic, Model 1191). The level of radioactivity directly corresponded to the extent of albumin binding to the polymer surface.

The preferred embodiments of this invention are better illustrated by the working examples which follow. Each example is a working representative of the respectively numbered reaction schemes illustrated above.

Example I

A 2 cm$^2$ section of 2 mm thick polyurethane sheet (Pellethane 2363—80A marketed by The UpJohn Company, Kalamazoo, Michigan) was soaked in redistilled toluene (Fisher, estimated purity 99%) to remove surface impurities. The sheet was then transferred to 25 ml of 0.04 M sodium ethoxide (sodium ethoxide was prepared with pure sodium and spectrograde ethanol both marketed by Aldrich) in toluene agitating under dry nitrogen at room temperature for fifteen minutes. In the same vessel 25 ml of 2.0 M 1-bromo-octodecane (reagent grade produced by Aldrich) was reacted with the polymer sample while mixing at ambient room temperature for fifteen minutes. The chemically derivatized sheet was then removed from the reaction vessel and consecutively soaked for 30 seconds at ambient room temperature as follows: 25 ml of toluene once, 25 ml of ethanol twice, 25 ml of deionized water twice, 25 ml of 0.1N hydrochloric acid once, and finally 25 ml of deionized water twice, prior to air drying for 24 hours. A polyurethane was obtained having random surface amine and N-octadecyl urethane substitutions. Subsequent albumin studies indicated up to 7-fold albumin binding enhancement as compared to control samples of non-alkyl-substituted polymer materials.

Example II

One gram portions of 4 mm diameter beads of Nylon® 6/10 and Nylon® 11 were washed separately in toluene and dried overnight, treated with sodium ethoxide and reacted with n-octadecyl bromide as described in Example I.

Example III

Fifty milligram portions of woven polyester (i.e., Dacron®) prosthetic vasculature were separately washed with toluene and dried overnight, treated with sodium ethoxide and reacted with octadecyl bromide as described in Example I. Increase of 30—50% in the binding of $^{125}$I-albumin was obtained upon 30 second incubation of the derivatized Dacron® with the radiolabelled albumin protein.

Example IV

Two hundred milligram portions of polyacrylamide beads (50—100 mesh size) were separately washed with toluene and dried overnight, treated with sodium ethoxide and reacted with octadecyl bromide as described in Example I.

Example V

A 1 cm$^2$ section of 2 mm thick polyurethane was soaked in toluene for 10 minutes and ethanol for 20 minutes. After vacuum drying overnight the section was placed in 50 ml of 0.25 M n-octadecylisocyanate in trimethylpentane under $N_2$ and incubated for one hour at 80°C with agitation. The sample was removed, twice soaked for 1 minute in 25 ml of ethanol, then twice soaked for 1 minute in 25 ml of deionized water before being redried to yield N-octadecyl-derivatized polyurethane. Radiolabelling studies of this derivatized polymer with $^{125}$I-albumin demonstrated up to a 5-fold enhancement in albumin binding.

Example IV

A one gram portion of 4 mm diameter Nylon® 6/12 beads was derivatized as described in Example V and yielded a similar 5-fold increase in the binding of $^{125}$I-albumin. Derivatization of Nylon® 11 beads behaved similarly.

Example VII

Fifty milligram portions of woven polyester (i.e., Dacron®) prosthetic vasculature were separately derivatized as described in Example V.

Example VIII

Two hundred milligram portions of polyacrylamide beads (50—100 mesh size) were separately derivatized as described in Example V.

Example IX

Twenty-five milligram portions of woven polyester (i.e., Dacron®) prosthetic vasculature were exposed to 2 ml of 0.02 M sodium ethoxide (NaOEt) dissolved in toluene:ethanol (3:1) for 1 hour at 55—60°C. After removing the NaOEt solution by aspiration, the "activated" portions were treated with 2 ml of 1.0 M

dodecyl primary amine dissolved in tertiary butyl alcohol ($^{t}$BuOH) containing 0.02 M triethylamine (TEA) for 2 hours at 20—25°C. These portions were eluted with ethanol (2 × 2 ml), 1%(v/v) acetic acid in methanol (2 × 2 ml), and again with ethanol (2 × 2 ml) before drying overnight *in vacuo* over $P_2O_5$. Upon radiolabelling exposure, there were increases of 30—50% in the binding of $^{125}$I-albumin as compared to non-substituted polyesters.

Utility

Alkyl-substituted polymers of the present invention are useful especially as construction materials for a wide variety of biomaterials. For example, these products are useful in the construction of vascular graft tubing, dialysis membranes and dialysis exchangers, microporous membrane blood oxygenators, intra-aortic balloons, ultrafiltration membranes, blood bags, various catheters, coatings for sutures, soft or hard tissue protheses, and artificial organs. Further, the alkyl-substituted polymers may have industrial applications in, for example, equilibrium dialysis.

Although the invention has been described in terms of particular embodiments which the Applicants believe to represent the best modes of the invention at the time of this application, it will be recognized by those skilled in the art that various changes may be made in the product and method embodiments of this specification without departing from the scope of the invention as defined in the following claims.

**Claims**

1. A biocompatible, nonthrombogenic polymeric material, characterized in that it comprises a polymeric substrate having covalently attached thereon aliphatic chains having from 14 to 30 carbon atoms, the chains providing selective, reversible affinity-binding sites for albumin.

2. A polymeric material according to claim 1, wherein the extensions have from 14 to 18 carbon atoms.

3. A polymeric material according to claim 1 or 2, characterized in that the polymer substrate is polyurethane, polyacrylamide, polyester, polyamine, polyimine, polystyrene, cellulose or polyamide.

4. A polymeric material according to claim 1, 2 or 3, characterized in that the aliphatic chains are straight-chained alkyl groups.

5. A polymeric material according to any one of the preceding claims, characterized in that the aliphatic chains are n-octadecylgroups.

6. A device adapted for use in contact with blood components containing albumin, characterized in that the device has disposed on the surfaces thereof which contact blood components a polymeric material in accordance with any one of claims 1 to 5.

7. A vacular graft comprising a tubular conduit, characterized in that the tubular conduit is constructed of a polymeric material in accordance with any one of claims 1 to 5.

8. A method of minimizing thrombus formation on the surfaces of a device adapted for use in the presence of blood components containing albumin, characterized in that the method comprises the step of providing a layer of polymeric material in accordance with any one of claims 1 to 5 on the surface of the device at the area of contact between the blood components and the device.

**Patentansprüche**

1. Biokompatibles, nicht-thrombogenes polymeres Material, dadurch gekennzeichnet, daß es ein polymeres Substrat mit kovalent daran gebundenen aliphatischen Ketten mit 14 bis 30 Kohlenstoffatomen enthält, wobei die Ketten selektive, reversible Affinitäts-Bindungsstellen für Albumin liefern.

2. Polymeres Material nach Anspruch 1, wobei die Ketten 14 bis 18 Kohlenstoffatome aufweisen.

3. Polymeres Material nach Anspruch 1 oder 2, dadurch gekennzeichnet, daß das Polymersubstrat Polyurethan, Polyacrylamid, Polyester, Polyamin, Polyimin, Polystyrol, Cellulose oder Polyamid ist.

4. Polymeres Material nach Anspruch 1, 2 oder 3, dadurch gekennzeichnet, daß die aliphatischen Ketten geradkettige Alkylgruppen sind.

5. Polymeres Material nach einem der vorangehenden Ansprüche, dadurch gekennzeichnet, daß die aliphatischen Ketten n-Octadecylgruppen sind.

6. Vorrichtung, die für eine Verwendung geeignet ist, bei der sie in Kontakt mit Blutbestandteilen kommt, die Albumin enthalten, dadurch gekennzeichnet, daß die Vorrichtung auf denjenigen Oberflächen, die in Kontakt mit Blutbestandteilen kommen, ein polymeres Material nach einem der Ansprüche 1 bis 5 trägt.

7. Vakulartransplantat, das einen röhrenförmigen Schlauch aufweist, dadurch gekennzeichnet, daß der röhrenförmige Schlauch aus einem polymeren Material nach einem der Ansprüche 1 bis 5 hergestellt ist.

8. Verfahren, um die Thrombusbildung auf der Oberfläche einer Vorrichtung, die für die Verwendung in der Gegenwart von Blutbestandteilen, die Albumin enthalten, geeignet ist, auf ein Mindestmaß zurückzuführen, dadurch gekennzeichnet, daß das Verfahren den Schritt enthält, eine Schicht aus polymerem Material nach einem der Ansprüche 1 bis 5 auf der Oberfläche der Vorrichtung in dem Bererich des Kontaktes zwischen den Blutbestandteilen und der Vorrichtung zur Verfügung zu stellen.

**Revendications**

1. Matière polymérique non thrombogénique biocompatible, caractérisée en ce qu'elle comprend un substrat polymérique auquel sont attachées par covalence des chaînes aliphatiques ayant 14 à 30 atomes de carbone, les chaînes procurant des sites sélectifs de liaison réversible par affinité pour l'albumine.

2. Matière polymérique suivant la revendication 1, dans laquelle les chaînes ont 14 à 18 atomes de carbone.

3. Matière polymérique suivant la revendication 1 ou 2, caractérisée en ce que le substrat polymérique est un polyuréthanne, un polyacrylamide, un polyester, une polyamine, une polyimine, un polystyrène, la cellulose ou un polyamide.

4. Matière polymérique suivant la revendication 1, 2 ou 3, caractérisée en ce que les chaînes aliphatiques sont des groupes alkyle à chaîne droite.

5. Matière polymérique suivant l'une quelconque des revendications précédentes, caractérisée en ce que les chaînes aliphatiques sont des groupes n-octadécyle.

6. Dispositif conçu pour être utilisé au contact de composants du sang contenant de l'albumine, caractérisé en ce qu'il porte sur ses surfaces qui entrent en contact avec des composants du sang, une matière polymérique suivant l'une quelconque des revendications 1 à 5.

7. Greffe vasculaire comprenant un conduit tubulaire, caractérisée en ce que le conduit tubulaire est réalisé en une matière polymérique suivant l'une quelconque des revendications 1 à 5.

8. Procédé pour minimiser la formation d'un thrombus sur les surfaces d'un dispositif conçu pour être utilisé en présence de composants du sang contenant de l'albumine, caractérisé en ce qu'il comprend l'étape qui consiste à doter d'une couche de matière polymérique suivant l'une quelconque des revendications 1 à 5 la surface du dispositif dans l'aire de contact entre les composants du sang et le dispositif.